# EUROPEAN PATENT SPECIFICATION

(11) **EP 4 452 154 B1**
(45) Date of publication and mention of the grant of the patent: **07.10.2026**
(21) Application number: 22840019.8
(22) Date of filing: 21.12.2022
(51) Int. Cl.: A61F 5/445

(54) **SENSOR PATCH FOR OSTOMY APPLIANCE**
SENSORPFLASTER FÜR STOMAVORRICHTUNG
PATCH DE CAPTEUR POUR APPAREIL DE STOMIE

(30) Priority: 21.12.2021 DK PA202170640
(43) Date of publication of application: 30.10.2024
(73) Proprietor: Coloplast A/S, 3050 Humlebaek (DK)
(72) Inventor: MOLZEN, Lars, 3450 Alleroed (DK); SUND, Anders Grove, 3450 Alleroed (DK); HANSEN, Kristoffer, 2850 Naerum (DK)
(86) International application number: PCT/DK2022/050304
(87) International publication number: WO 2023/117022

(56) References cited:
- WO-A1-2007/098762
- WO-A1-2020/252458
- US-A1- 2020 246 175

## Description

The present disclosure relates to a medical device, and in particular to a sensor patch for an ostomy appliance for attachment to the skin surface of a user. In particular, the present disclosure relates to a sensor patch for attachment to the adhesive surface of a base plate, the sensor patch comprising a nonwoven substrate and electrodes for sensing moisture.

### Brief description of the drawings

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Fig. 1 illustrates an exploded perspective view of an exemplary sensor patch according to an embodiment of the invention;
Fig. 2 illustrates an exploded side view of an exemplary sensor patch according to an embodiment of the invention;
Fig. 3 illustrates an exploded side view of an exemplary sensor patch according to an embodiment of the invention; and
Fig. 4 illustrates liquid transport through an exemplary sensor patch.

### Background

WO 2007/098762 A1 relates to a method and dressing for detecting detachment of the dressing, which is applied to a surface of an at least partly electrically conductive object. The dressing comprises an adhesive and at least two electrodes arranged in a distance from the electrically conductive object. Changes in capacitance are detected, and an alarm is activated when the changes of the capacitance reach a predetermined value.

### Detailed description

In the following the invention is described in further detail. It is however noted that none of the methods mentioned fall under the scope of protection.

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

Throughout this disclosure, the words "stoma" and "ostomy" are used to denote a surgically created opening bypassing the intestines or urinary tract system of a person. The words are used interchangeably, and no differentiated meaning is intended. The same applies for any words or phrases derived from these, e.g. "stomal", "ostomies" etc. Also, the solid and liquid wastes emanating from the stoma may be referred to as both stomal "output," "waste(s)," "liquids," and "fluids" interchangeably. A subject having undergone ostomy surgery may be referred to as "ostomist" or "ostomate" - moreover, also as "patient" or "user". However, in some cases "user" may also relate or refer to a health care professional (HCP), such as a surgeon or an ostomy care nurse or others. In those cases, it will either be explicitly stated, or be implicit from the context that the "user" is not the "patient" him- or herself.

In the following, whenever referring to proximal side or surface of a layer, an element, a device or part of a device, the referral is to the skin-facing side or surface, when a user wears the ostomy appliance. Likewise, whenever referring to the distal side or surface of a layer, an element, a device or part of a device, the referral is to the side or surface facing away from the skin, when a user wears the ostomy appliance. In other words, the proximal side or surface is the side or surface closest to the user, when the appliance is fitted on a user and the distal side is the opposite side or surface - the side or surface furthest away from the user in use.

The axial direction is defined as the direction of the stoma, when a user wears the appliance. Thus, the axial direction is generally perpendicular to the skin or abdominal surface of the user.

A radial direction is defined as perpendicular to the axial direction. In some sentences, the words "inner" and "outer" may be used. These qualifiers should generally be perceived with respect to the radial direction, such that a reference to an "outer" element means that the element is farther away from a centre portion of the ostomy appliance than an element referenced as "inner". In addition, "innermost" should be interpreted as the portion of a component forming a centre of the component and/or being adjacent to the centre of the component. In analogy, "outermost" should be interpreted as a portion of a component forming an outer edge or outer contour of a component and/or being adjacent to that outer edge or outer contour.

The use of the word "substantially" as a qualifier to certain features or effects in this disclosure is intended to simply mean that any deviations are within tolerances that would normally be expected by the skilled person in the relevant field.

The use of the word "generally" as a qualifier to certain features or effects in this disclosure is intended to simply mean - for a structural feature: that a majority or major portion of such feature exhibits the characteristic in question, and - for a functional feature or an effect: that a majority of outcomes involving the characteristic provide the effect, but that exceptionally outcomes do no provide the effect.

The present disclosure provides a sensor patch for attachment to the adhesive surface of a base plate of an ostomy appliance.

The present disclosure relates to an ostomy system and devices thereof, such as an ostomy appliance, a base plate for an ostomy appliance, a sensor patch for application to a base plate, a monitor device, and optionally one or more accessory devices. Further, methods related to the ostomy system and devices thereof are disclosed. Said methods do however not form part of the scope of protection. An accessory device (also referred to as an external device) can be a mobile phone or other handheld device, such as a smart device including a smartphone or a smartwatch. In embodiments, an accessory device is a personal electronic device, e.g., a wearable, such as a watch or other wrist-worn electronic device. An accessory device can be a docking station. In embodiments, the docking station is configured to electrically and/or mechanically couple the monitor device to the docking station. In embodiments, the docking station is configured for charging a battery of the monitor device and/or configured for transferring data between the monitor device and the docking station. The ostomy system can comprise a server device. In embodiments, the server device is operated and/or controlled by the ostomy appliance manufacturer and/or a service centre.

The present disclosure provides an ostomy system and devices thereof, such as an ostomy appliance, a base plate for an ostomy appliance, a sensor patch for application to a base plate, a monitor device, and optionally one or more accessory devices which either alone or together facilitate reliable determination of the nature, severity, and rapidness of moisture propagation in the adhesive material provided for attaching the base plate and/or sensor patch to the skin surface of a user. Depending on the nature of the pattern of moisture propagation in the adhesive, the ostomy system and devices thereof enable providing information to the user about the type of failure, such as adhesive failure patterns, and in turn enable providing an indication to the user of the severity and thus the remaining time frame for replacing the ostomy appliance without experiencing severe leakage and/or skin damage.

In embodiments, the ostomy appliance includes a base plate, such as a monolithic, one-piece base plate, e.g., integrated with a sensor assembly part, or a separate sensor assembly part, such as a sensor assembly part to be subsequently applied to a base plate. In embodiments, the sensor assembly part is a sensor patch for application to the base plate, such as the proximal surface of the base plate. Thereby, an arbitrary base plate, such as a conventional base plate provided with the sensor patch, can achieve the features as described herein. Features as described with respect to sensing/monitoring capabilities of the base plate herein can be provided by a sensor assembly of a sensor patch to be applied to a base plate, e.g., by the user, and vice versa. In embodiments, the sensor patch is adapted to adhere to a base plate. In embodiments, the sensor patch comprises am adhesive layer adapted to adhere to the skin surface of a user.

In embodiments, a method (which does not form part of the scope of protection) of attaching a base plate having sensing capabilities, e.g., through the provision of a sensor patch, to a user's stoma and/or skin surrounding the stoma, such as the peristomal skin area, comprises attaching the sensor patch to a base plate and attaching the base plate, i.e., together with the attached sensor patch, to the user's stoma and/or skin surrounding the stoma, such as the peristomal skin area. Alternatively, the method of attaching the base plate to the user's stoma and/or skin surrounding the stoma comprises attaching the sensor patch to the user's stoma and/or skin surrounding the stoma and attaching the base plate to the user's stoma and/or skin surrounding the stoma above the attached sensor patch, i.e., on a distal surface of the sensor patch.

In embodiments, the ostomy appliance comprises a base plate and an ostomy pouch (also referred to as an ostomy bag). The ostomy appliance can be a colostomy appliance, an ileostomy appliance, or a urostomy appliance. In embodiments, the ostomy appliance is a two-part ostomy appliance, i.e., the base plate and the ostomy pouch are releasably coupled, e.g., with a mechanical and/or an adhesive coupling, e.g., to allow that a plurality of ostomy pouches can be utilized (exchanged) with one base plate. Further, a two-part ostomy appliance can facilitate correct application of the base plate to skin, e.g., to an improved user sight of the stomal region. In embodiments, the ostomy appliance is a one-part ostomy appliance, i.e., the base plate and the ostomy pouch are fixedly attached to each other. The base plate is configured for coupling to a user's stoma and/or skin surrounding the stoma, such as a peristomal skin area.

In embodiments, the ostomy system comprises a base plate or a sensor patch for application to a base plate. In embodiments, parts of the ostomy system are incorporated into a base plate or a sensor patch for application to a base plate. In embodiments, an adhesive layer of the ostomy system is an adhesive layer of a base plate or a sensor patch for attachment to a base plate. In embodiments, the adhesive layer and the sensor assembly of the ostomy system are incorporated into a base plate or sensor patch to provide such with the ability to indicate a site of leakage. In embodiments, the ostomy system provides a base plate and/or a sensor patch with the ability to indicate a site of leakage from an ostomy appliance, e.g., from the base plate.

Providing a base plate having sensing capabilities, e.g., through an incorporated sensor assembly or through a sensor patch comprising a sensor assembly provides for an optimum or improved use of an ostomy appliance. In particular, it is facilitated that a base plate is not changed too late (leading to adhesive failure, leakage, and/or skin damage), or at least that a user is informed that a leakage will happen, is happening, or has happened. Accordingly, the user or a health care professional is able to monitor and plan the use of the ostomy appliance.

In a first aspect of the invention, a sensor patch is disclosed, the sensor patch being for attachment to the adhesive surface of a base plate of an ostomy appliance.

In embodiments, the sensor patch has a proximal surface for attachment to the skin surface of a user and a distal surface for the attachment of the sensor patch to the adhesive surface of a base plate. The sensor patch comprises:
- an adhesive layer having a proximal side including a proximal surface for adhering the sensor patch to the skin, and a distal side including a distal surface;
- a permeable nonwoven substrate arranged on the distal side of the adhesive layer, the nonwoven substrate having a proximal side including a proximal surface and a distal side including a distal surface; and
- an electrode assembly arranged on the proximal surface of the nonwoven substrate.

In embodiments, the sensor patch has a proximal surface for attachment to the skin surface of a user and a distal surface for the attachment of the sensor patch to the adhesive surface of a base plate. The sensor patch comprises:
- an adhesive layer having a proximal side including a proximal surface for adhering the sensor patch to the skin, and a distal side including a distal surface; and
- a sensing layer comprising a nonwoven substrate and electrical conductors, the sensing layer being arranged on the distal side of the adhesive layer, the sensing layer having a proximal side including a proximal surface and a distal side including a distal surface, the sensing layer being liquid permeable, the nonwoven substrate having a proximal side including a proximal surface and a distal side including a distal surface.

By liquid permeable is meant liquid and/or moisture permeable.

Thereby is provided a sensor patch with improved permeability towards a base plate onto which it is to be attached, whereby liquid/moisture may be absorbed by the adhesive layer of the sensor patch and due to the permeable nature of the nonwoven substrate, said liquid/moisture may propagate further into the base plate adhesive. Thereby, the base plate adhesive may be utilized.

Further, the sensor patch provides for a sensing accessory for a base plate with improved flexibility due to the nonwoven material, as will be discussed in greater detail below.

In embodiments, the electrical conductors are integrated into the nonwoven substrate.

In embodiments, the electrical conductors may comprise an electrode assembly, or the electrical conductors may form an electrode assembly, or form part of an electrode assembly. In the following, "electrode assembly" may be used to denote the electrical conductors. The electrical conductors may be denoted electrodes. The electrical conductors may be denoted electrodes of the electrode assembly.

The nonwoven substrate provides a liquid permeable material that is at least partly electrical conducting.

In embodiments, the electrical conductors are arranged on the proximal surface of the nonwoven substrate.

In embodiments, the electrical conductors are arranged on the distal surface of the nonwoven substrate.

Thus, the sensor patch is capable of detecting liquid in the adhesive layer and/or the nonwoven substrate.

In embodiments, the electrical conductors are printed traces of conducting material, printed on the nonwoven substrate. The printed traces may be printed such that they are positioned on one side of the nonwoven substrate or such that the printed traces are positioned on one side of the nonwoven substrate and partly inside the substrate or the printed traces may extend from one side of the nonwoven substrate through the substrate and to the other side of the substrate. The parts of the sensing layer that is conductive may be liquid permeable.

In embodiments, at least a part of the sensing layer comprises a nonwoven substrate.

In embodiments, the sensing layer comprises a nonwoven substrate and/or a polymeric substrate.

Thereby is provided a sensor patch with electrodes that may be at least partly liquid permeable for detecting the liquid absorbed in the sensor patch while liquid and/or moisture can be transported to a base plate to be arranged on the distal side of the sensor patch for giving a larger capacity of liquid absorption.

In embodiments, the adhesive layer, such as a first adhesive layer, is made of a first composition. In embodiments, the first composition comprises one or more polyisobutenes and/or styrene-isoprene-styrene. In embodiments, the first composition comprises one or more hydrocolloids. In embodiments, the first composition comprises one or more water soluble or water swellable hydrocolloids. In embodiments, the first composition is a pressure sensitive adhesive composition suitable for medical purposes comprising a rubbery elastomeric base and one or more water soluble or water swellable hydrocolloids. In embodiments, the first composition comprises one or more polybutenes, one or more styrene copolymers, one or more hydrocolloids, or any combination thereof. The combination of the adhesive properties of the polybutenes and the absorbing properties of the hydrocolloids renders the first composition suitable for use in ostomy appliances. For example, the styrene copolymer can be a styrene-butadiene-styrene block copolymer or a styrene-isoprene-styrene block copolymer. Preferably, one or more styrene-isoprene-styrene (SIS) block type copolymers are employed. The amount of styrene blockcopolymer can be from 5% to 20% of the total adhesive composition. The butene component is suitably a conjugated butadiene polymer selected from polybutadiene, polyisoprene. The polybutenes are preferably present in an amount of from 35 - 50% of the total adhesive composition. Preferably, the polybutene is polyisobutylene (PIB). Suitable hydrocolloids for incorporation in the first composition are selected from naturally occurring hydrocolloids, semisynthetic hydrocolloids, and synthetic hydrocolloids. The first composition can comprise 20-60% hydrocolloids. A preferred hydrocolloid is carboxymethyl cellulose (CMC). Optionally, the first composition can contain other components, such as fillers, tackifiers, plasticizers, and/or other additives.

The adhesive layer can have a substantially uniform thickness. The adhesive layer can have a thickness in the range from 0.08 mm to 1.5 mm, e.g., in the range from 0.1 mm to 0.5 mm, thereby providing a thin adhesive, such that moisture may quickly reach the nonwoven substrate. Alternatively, the adhesive layer may have a thickness in the range between 0.5 mm and 1.5 mm, such as between 0.8 mm and 1.0 mm. The adhesive layer can have a primary thickness in a primary part of the adhesive layer, e.g., in a primary region within a primary radial distance or in a primary radial distance range from the centre point of the stomal opening. The primary radial distance can be in the range from 20 mm to 50 mm, such as in the range from 25 mm to 35 mm, e.g., 30 mm. The adhesive layer can have a secondary thickness in a secondary part of the adhesive layer, e.g., in a secondary region outside a secondary radial distance or in a secondary radial distance range from the centre point of the stomal opening. The secondary radial distance can be in the range from 20 mm to 50 mm, such as in the range from 25 mm to 35 mm, e.g., 30 mm. The secondary thickness may be smaller than the primary thickness.

The ostomy appliance comprises a nonwoven substrate arranged on the distal side of the adhesive layer. By a nonwoven substrate is meant a nonwoven textile fabric, i.e., a textile comprising a plurality of intertwined fibres made according to common nonwoven fabrication techniques, such as spunlaid/spunbond nonwovens or melt-blown nonwovens, although the use of other types of nonwovens are foreseen. In other words, by a nonwoven fabric or textile is meant a material made from fibres, such as stable fibres and/or long fibres, bonded together (entangled) by chemical, mechanical, heat or solvent treatment.

For example, the nonwoven substrate is arranged on the distal surface of the adhesive layer. Since the adhesive layer is moisture absorbing, such moisture will eventually reach the nonwoven substrate arranged on the distal side of said adhesive layer. Here, the moisture may come into contact with the electrodes of the electrode assembly, thereby facilitating the sensing of moisture.

In embodiments, the fibres of the nonwoven substrate may be natural fibres. In embodiments, the fibres of the nonwoven substrate may be synthetic fibres. In embodiments, the nonwoven substrate comprises synthetic fibres made of one or more of polypropylene (PP), polyester (PE), polyurethane (PU), polyethylene terephthalate (PET), polyethersulfone (PES) and polyamide/nylon (PA). For example, the nonwoven substrate may comprise one or more (a blend) of said synthetic fibres.

In embodiments, the nonwoven substrate has a weight (area density) between 10 g/m² and 40 g/m². The weight/area density may be measured according to ISO 9073-1. In embodiments, the nonwoven substrate has a thickness between 75 µm and 270 µm. The thickness may be measured according to ISO 9073-2.

In an embodiment, the nonwoven substrate is moisture permeable to facilitate a transfer of moisture from the proximal side of the nonwoven substrate to the distal side of the nonwoven substrate. In a preferred embodiment, the nonwoven substrate is moisture and/or liquid permeable.

Thereby, moisture in the adhesive layer of the sensor patch may permeate through the nonwoven substrate (e.g., from the proximal side of the nonwoven substrate to the distal side of the nonwoven substrate) and further get absorbed by an adhesive layer of a base plate onto which the sensor patch may be adhered/attached. Thereby, the nonwoven substrate facilitates utilization/activation of the moisture absorbing capacity of the base plate layered with the sensor patch.

Electrical conductors, such as part of an electrode assembly, may be arranged on a surface of the nonwoven substrate. For example, the electrode assembly comprises one or more, such as two or more, electrodes printed on a surface, such as the proximal or distal surface, of the nonwoven substrate. The electrodes of the electrode assembly are provided on the nonwoven substrate in such a way that signals (current) may be conducted through said electrodes, whereby the electrode assembly may facilitate detection of presence of moisture, such as moisture absorbed by the adhesive layer. For example, the moisture may be due to liquid in the interface between the skin surface and the proximal side of the adhesive layer. For example, the liquid may be sweat and/or output from the stoma.

In an embodiment, the sensor patch comprises a monitor interface for electrically coupling a monitor device to the electrode assembly/electrical conductors. The monitor interface may comprise coupling means for detachably fixating a monitor device relative to the monitor interface. The monitor interface is coupled to the electrode assembly, such that a monitor device coupled to the monitor interface may use electrodes of the electrode assembly to detect presence of liquid. The monitor interface may comprise a plurality of terminals coupled to electrodes of the electrode assembly, the terminals being configured to electrically couple with corresponding terminals of the monitor device.

The electrodes and/or the terminals may comprise one or more of metallic (e.g., silver, copper, gold, titanium, aluminium, stainless steel), ceramic (e.g., ITO), polymeric (e.g., PEDOT, PANI, PPy), and carbonaceous (e.g., carbon black, carbon nanotube, carbon fibre, graphene, graphite) materials.

In an embodiment, the distal surface of the nonwoven substrate is the distal surface of the sensor patch. In an embodiment, the distal surface of the nonwoven substrate is configured to adhere to the adhesive surface of a base plate of an ostomy appliance. Thereby, the distal surface of the nonwoven substrate is configured for attachment to an adhesive surface of a base plate of an ostomy appliance. For example, the user may attach the sensor patch to the adhesive surface of a base plate by him-/herself, i.e., at home during the application procedure.

In an embodiment, the electrode assembly comprises a plurality of electrodes. In embodiments, the plurality of electrodes may be denoted a plurality of electrical conductors. In an embodiment, the plurality of electrodes includes a first electrode and a second electrode, such as a first electrical conductor and a second electrical conductor. The plurality of electrodes may further comprise a third, a fourth, a fifth, a sixth, a seventh and/or an eight electrode (electrical conductors). Thereby, the electrodes of the electrode assembly may provide for detecting moisture in the adhesive layer. For example, a monitor device coupled to the sensor patch may be configured to apply a voltage across the first and the second electrode and thereby determine an electrical quantity, e.g., resistance, impedance, capacitance and/or conductance. The electrical quantity may be indicative of moisture in the adhesive layer. For example, moisture in the adhesive layer may cause the resistance to decrease.

In an embodiment, the plurality of electrodes is conductive traces printed on the proximal surface of the nonwoven substrate. For example, electrodes may be printed with a conductive ink, thereby resulting in conductive traces on the nonwoven substrate upon curing.

In a second aspect of the invention, an ostomy system is disclosed. The ostomy system comprises a sensor patch according to the first aspect of the invention and a base plate of an ostomy appliance. The base plate has an adhesive layer with an adhesive surface configured for attachment to the skin surface of a user, and the distal surface of the sensor patch is configured for attachment to the adhesive surface of the base plate. The sensor patch may be configured to be adhered to the adhesive surface of the base plate by the user him-/herself. Thereby, the base plate may be provided with the sensing abilities of the sensor patch, and due to the provision of the permeable nonwoven substrate of the sensor patch, moisture may be transported through the adhesive layer of the sensor patch, through the nonwoven substrate and into the adhesive of the base plate. The adhesive of the base plate may be made of a second composition being substantially identical to the first composition as previously disclosed. For example, the second composition may comprise liquid absorbing particles, such as hydrocolloids. The second composition may differ from the first composition in the ratio of the contents. For example, different ratio of contents can change properties of the first and/or second compositions, thereby giving the adhesive layer and the adhesive of the base plate different properties. For example, the adhesive layer of the base plate may be optimised for absorbing liquid having permeated through the sensor patch.

### Detailed description of the drawings

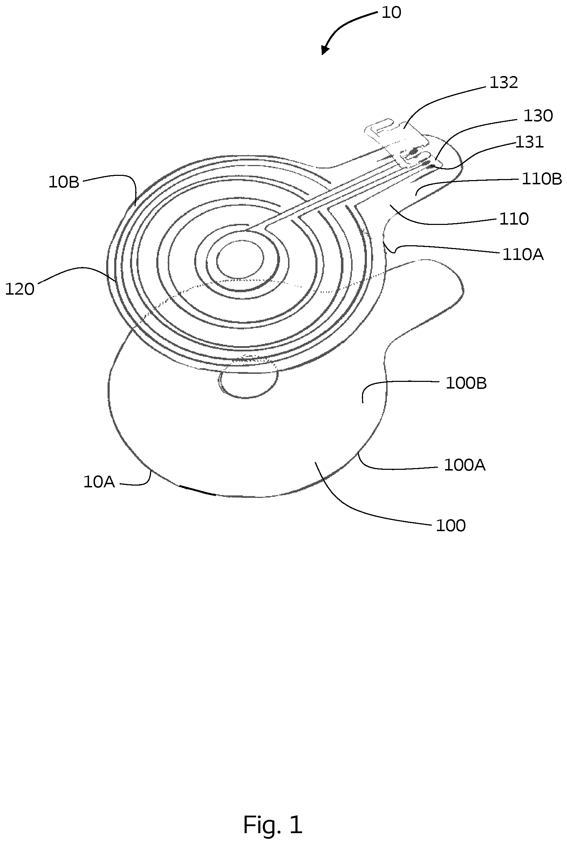

Fig. 1 illustrates an exploded perspective view of an exemplary sensor patch 10 according to an embodiment of the invention. The sensor patch 10 has a proximal surface 10A for attachment to the skin surface of a user and a distal surface 10B for the attachment of the sensor patch 10 to the adhesive surface of a base plate (not shown). The sensor patch 10 comprises:
- an adhesive layer 100 having a proximal side including a proximal surface 100A for adhering the sensor patch 10 to the skin, and a distal side including a distal surface 100B;
- a permeable nonwoven substrate 110 arranged on the distal side of the adhesive layer 100, the nonwoven substrate 110 having a proximal side including a proximal surface 110A and a distal side including a distal surface 110B; and
- an electrode assembly 120 arranged on the proximal surface 110A of the nonwoven substrate 110.

In the illustrated embodiment, the nonwoven substrate 110 is transparent, thereby highlighting the electrode assembly 120 arranged on the proximal surface 110A of the nonwoven substrate 110.

The electrode assembly 120 may comprise or consist of electrical conductors.

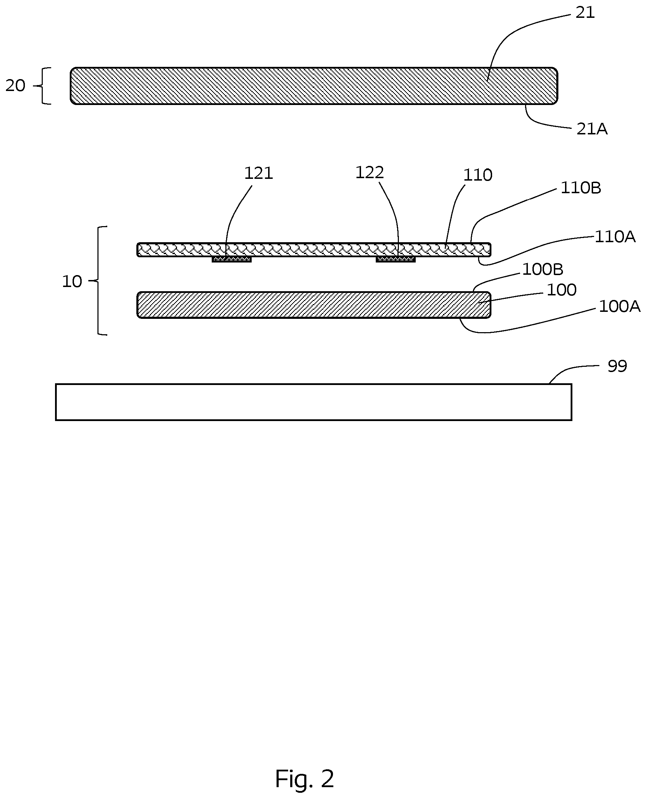

The electrode assembly 120 may comprise a plurality of electrodes (electrical conductors) as illustrated in Fig. 2, such as a first electrode 121 and a second electrode 122. The electrodes may be printed with a conductive ink, thereby forming conductive traces on the nonwoven substrate 110. The electrode assembly 120 facilitate detection of liquid/moisture absorbed by the adhesive layer 100, in particular when coupled with a monitor device (not shown) via the monitor interface 130 comprising a plurality of terminals 131.

The monitor interface 130 facilitates a coupling, such as an electrical and mechanical coupling, between the sensor patch 10 and a monitor device comprising a processor, a memory, a power unit and at least one interface for the coupling with the monitor interface 130 of the sensor patch 10. The monitor interface 130 may comprise a connector 132 to facilitate a fixated and releasable coupling with a monitor device.

The distal surface 110B of the nonwoven substrate 110 may be the distal surface 10B of the sensor patch 10 as such. Thereby, the nonwoven substrate 110 may be adapted to be adhered/attached directly to a proximal adhesive surface of a conventional base plate (not shown), such as by the user him-/herself. The proximal surface 100A of the adhesive layer 100 may be protected with a release liner (not shown) prior to application to the skin surface.

The nonwoven substrate 110 is permeable, such as liquid and/or moisture permeable. Thereby, when the distal surface 110B of the nonwoven substrate 110 is attached to a base plate adhesive (see reference 21 in Fig. 2), moisture absorbed by the adhesive layer 100 of the sensor patch 10 may permeate through the nonwoven substrate 110 and further into said base plate adhesive. Thereby, the absorbing capacity of the base plate adhesive may be utilized.

The nonwoven substrate 110 may be made of natural and/or synthetic fibres. The nonwoven substrate may comprise a blend of fibres of different properties and/or materials.

Fig. 2 illustrates an exploded side view of an exemplary sensor patch 10 according to an embodiment of the invention. Also shown is the skin surface 99 and a conventional base plate 20 with a base plate adhesive 21 having a proximal surface 21A. Thus, as illustrated, the sensor patch 10 is configured to be layered in the interface between the skin surface 99 and the proximal surface 21A of the base plate adhesive 21 of the conventional base plate 20. The base plate 20 may comprise coupling means for coupling an ostomy bag to the base plate, or the base plate 20 may comprise an ostomy bag. Not shown is the stomal opening. For example, the side view illustrated in Fig. 2 may illustrate a left-hand or right-hand side of the sensor patch 10 and base plate 20 relative to a stoma.

The sensor patch 10 comprises the adhesive layer 100 having a proximal surface 100A and a distal surface 100B and a nonwoven substrate 110 having a proximal surface 110A and distal surface 110B.

An electrode assembly or electrical conductors, here illustrated as a first electrode 121 and a second electrode 122 of the electrode assembly, is arranged on the proximal surface 110A of the nonwoven substrate 110, such as prior to assembly with the adhesive layer 100. Thus, the nonwoven substrate 110, with the electrode assembly, may subsequently be arranged on the distal surface 100B of the adhesive layer 100, such that the electrode assembly (the first electrode 121 and the second electrode 122) is sandwiched between the nonwoven substrate 110 and the adhesive layer 100. Thereby, the electrodes are exposed to the adhesive layer 100 and may, via a monitor device (not shown) coupled to the sensor patch, facilitate detection of moisture absorbed by the adhesive layer from the proximal surface 100A of said adhesive layer 100. For example, the electrodes 121,122 may be configured to measure the resistance of the adhesive layer, e.g., by means of a voltage applied across said electrodes by means of a monitor device coupled to the electrode assembly. Decreasing resistance may be indicative of moisture absorption. Similarly, the monitor device may measure impedance, conductance or capacitance to determine moisture absorption.

For example, the moisture may be liquid resulting from sweating or it may be output from the stoma propagating in the interface between the skin surface 99 and the adhesive layer 100 of the sensor patch 10 and/or the conventional base plate 20.

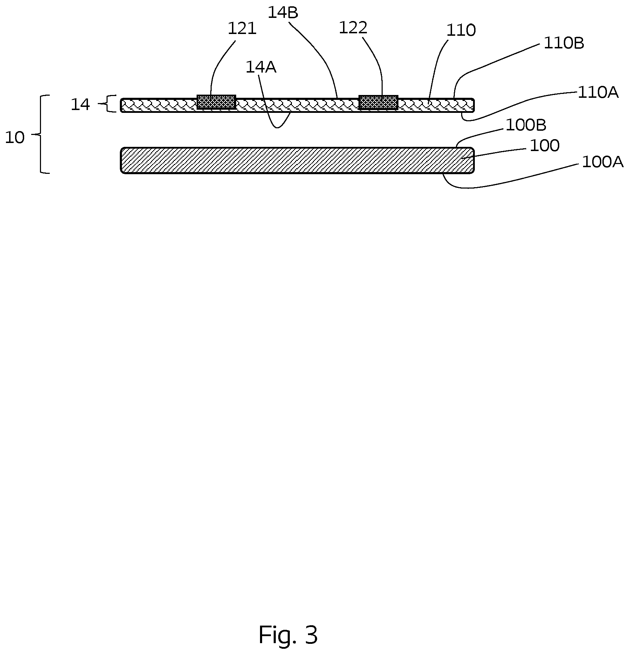

Fig. 3 illustrates an exploded view of a cross-section of a sensor patch 10. The sensor patch illustrated comprises an adhesive layer 100 and a sensing layer 14. The adhesive layer 100 has a proximal side 100A for contacting the skin and a distal side 100B. The sensing layer 14 is positioned on the distal side 100B of the adhesive layer 100. The sensing layer 14 has a proximal side 14A and a distal side 14B. The proximal side 14A of the sensing layer 14 faces the adhesive layer 100 and thus the skin. The sensing layer 14 comprises nonwoven substrate 110 and one or more electrical conductors 121, 122. The electrical conductors 121, 122 may be arranged on a side of the nonwoven layer 110, such as on the proximal surface 110A of the nonwoven layer 110, on the distal surface 110B of the nonwoven layer 110. The electrical conductors 121, 122 may be integrated into the nonwoven layer 110 either partly or fully. By having electrodes integrated in the nonwoven layer 110 the moisture or liquid transported into the nonwoven material or adhesive layer 100 can be detected.

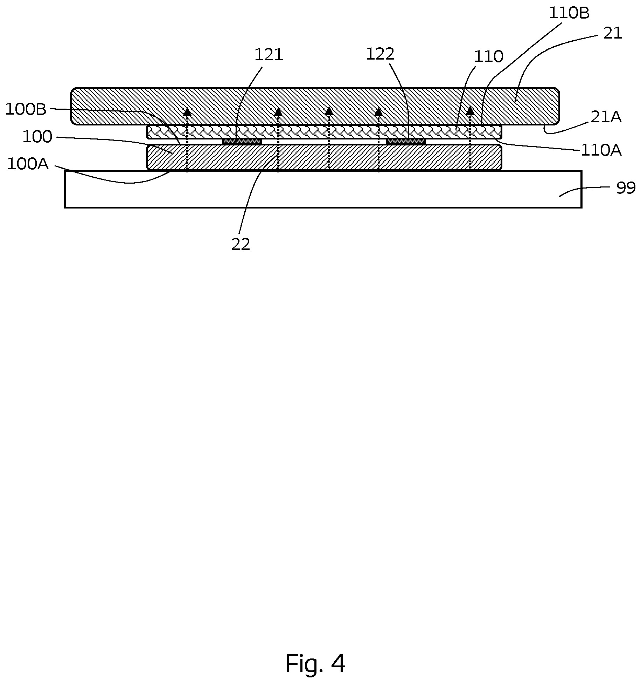

Fig. 4 illustrates how the moisture and liquid may be transported through the sensor patch. The sensor patch is illustrated in contact with the skin 99 and a proximal surface 21A of a base plate adhesive 21. Moisture and liquid may originate from the interface between the sensor patch and the skin 99, such as caused by sweat or stomal output from the stoma and propagating in the interface. Liquid at the interface between the sensor patch and the skin 99 may be absorbed by the adhesive layer 100 and transported to the interface between the adhesive layer 100 and the sensing layer. The sensing layer may further absorb the liquid in the non-woven material 110 and the liquid may be absorbed by the base plate 21. Thus, the dashed arrows 22 illustrate a liquid transport through the system.

Thus, the disclosed sensor patch 10 provides for (i) moisture detection by means of the electrode assembly 120 (e.g., electrical conductors) and (ii) for utilization of the moisture absorbing capacity of the base plate adhesive 21.

Although particular features have been shown and described, it will be understood that they are not intended to limit the claimed invention, and it will be made obvious to those skilled in the art that various changes and modifications may be made without departing from the scope of the claimed invention. For example, it is envisioned that the sensor assembly and the electrodes thereof may be incorporated and/or provided in a variety of different embodiments without departing from the scope of the invention.

The specification and drawings are, accordingly, to be regarded in an illustrative rather than restrictive sense. The claimed invention is intended to cover all alternatives, modifications, and equivalents.

## Claims

1. A sensor patch (10) for attachment to the adhesive surface (21A) of a base plate (20) of an ostomy appliance, the sensor patch having a proximal surface (10A) for attachment to the skin surface (99) of a user and a distal surface (10B) for attachment of the sensor patch to the adhesive surface (21A) of a base plate (20), the sensor patch comprising:
- an adhesive layer (100) having a proximal side including a proximal surface (100A) for adhering the sensor patch to the skin, and a distal side including a distal surface (100B); and
- a sensing layer (14) comprising electrical conductors (121, 122), the sensing layer arranged on the distal side of the adhesive layer (100), the sensing layer having a proximal side including a proximal surface and a distal side including a distal surface, the sensing layer being liquid permeable, and
**characterised in that** the sensing layer (14) further comprises a nonwoven substrate (110).

2. The sensor patch according to claim 1, wherein the electrical conductors are integrated into the nonwoven substrate.

3. The sensor patch according to claim 1, wherein the electrical conductors are arranged on a proximal surface (110A) of the nonwoven substrate.

4. The sensor patch according to claim 1, wherein the electrical conductors are arranged on a distal surface (110B) of the nonwoven substrate.

5. The sensor patch according to any of claims 1-4, wherein a distal surface (110B) of the sensing layer is the distal surface (100B) of the sensor patch.

6. The sensor patch according to claim 5, wherein the distal surface of the sensing layer is configured to adhere to the adhesive surface of a base plate of an ostomy appliance.

7. The sensor patch according to any of claims 1-6, wherein the electrical conductors comprise a plurality of electrodes, wherein the plurality of electrodes includes a first electrode (121) and a second electrode (122).

8. The sensor patch according to claim 7, wherein the plurality of electrodes are conductive traces printed on a surface of the nonwoven substrate.

9. The sensor patch according to any of claims 1-8, wherein the sensor patch comprises a monitor interface for electrically coupling a monitor device to the electrical conductors.

10. An ostomy system comprising a sensor patch (10) according to any of claims 1-9 and a base plate (20) of an ostomy appliance, wherein the distal surface of the sensor patch is configured for attachment to an adhesive surface of the base plate.

## Patentansprüche

1. Sensorpflaster (10) zum Anbringen an der Klebefläche (21A) einer Trägerplatte (20) einer Stomavorrichtung, wobei das Sensorpflaster eine proximale Oberfläche (10A) zum Anbringen an der Hautoberfläche (99) eines Benutzers und eine distale Oberfläche (10B) zum Anbringen des Sensorpflasters an der Klebefläche (21A) einer Trägerplatte (20) aufweist, wobei das Sensorpflaster Folgendes umfasst:
- eine Klebeschicht (100) mit einer proximalen Seite, die eine proximale Oberfläche (100A) zum Anbringen des Sensorpflasters an der Haut umfasst, und mit einer distalen Seite, die eine distale Oberfläche (100B) umfasst, und
- eine Erfassungsschicht (14), die elektrische Leiter (121, 122) umfasst, wobei die Erfassungsschicht auf der distalen Seite der Klebeschicht (100) angeordnet ist, wobei die Erfassungsschicht eine proximale Seite mit einer proximalen Oberfläche und eine distale Seite mit einer distalen Oberfläche aufweist, wobei die Erfassungsschicht flüssigkeitsdurchlässig ist, und
**dadurch gekennzeichnet, dass** die Erfassungsschicht (14) ferner ein Vliesstoffträgermaterial (110) umfasst.

2. Sensorpflaster nach Anspruch 1, wobei die elektrischen Leiter in das Vliesstoffträgermaterial integriert sind.

3. Sensorpflaster nach Anspruch 1, wobei die elektrischen Leiter auf einer proximalen Oberfläche (110A) des Vliesstoffträgermaterials angeordnet sind.

4. Sensorpflaster nach Anspruch 1, wobei die elektrischen Leiter auf einer distalen Oberfläche (110B) des Vliesstoffträgermaterials angeordnet sind.

5. Sensorpflaster nach einem der Ansprüche 1 bis 4, wobei eine distale Oberfläche (110B) der Erfassungsschicht die distale Oberfläche (100B) des Sensorpflasters ist.

6. Sensorpflaster nach Anspruch 5, wobei die distale Oberfläche der Erfassungsschicht dazu ausgelegt ist, an der Klebefläche einer Trägerplatte einer Stomavorrichtung zu haften.

7. Sensorpflaster nach einem der Ansprüche 1 bis 6, wobei die elektrischen Leiter eine Vielzahl von Elektroden umfassen, wobei die Vielzahl von Elektroden eine erste Elektrode (121) und eine zweite Elektrode (122) umfasst.

8. Sensorpflaster nach Anspruch 7, wobei es sich bei der Vielzahl von Elektroden um Leiterbahnen handelt, die auf einer Oberfläche des Vliesstoffträgermaterials aufgedruckt sind.

9. Sensorpflaster nach einem der Ansprüche 1 bis 8, wobei das Sensorpflaster eine Monitorschnittstelle zum elektrischen Koppeln einer Monitorvorrichtung mit den elektrischen Leitern umfasst.

10. Stomasystem, umfassend ein Sensorpflaster (10) nach einem der Ansprüche 1 bis 9 und eine Trägerplatte (20) einer Stomavorrichtung, wobei die distale Oberfläche des Sensorpflasters zum Anbringen an einer Klebefläche der Trägerplatte ausgelegt ist.

## Revendications

1. Patch de capteur (10) destiné à être fixée à la surface adhésive (21A) d'une plaque de base (20) d'un appareil de stomie, le patch de capteur présentant une surface proximale (10A) destinée à être fixée à la surface cutanée (99) d'un utilisateur et une surface distale (10B) pour la fixation du patch de capteur à la surface adhésive (21A) d'une plaque de base (20), le patch de capteur comprenant :
- une couche adhésive (100) ayant un côté proximal comprenant une surface proximale (100A) destiné à faire adhérer le patch de capteur à la peau, et un côté distal comprenant une surface distale (100B) ; et
- une couche de détection (14) comprenant des conducteurs électriques (121, 122), la couche de détection étant agencée du côté distal de la couche adhésive (100), la couche de détection ayant un côté proximal comprenant une surface proximale et un côté distal comprenant une surface distale, la couche de détection étant perméable au liquide, et
**caractérisé en ce que** la couche de détection (14) comprend en outre un substrat non tissé (110).

2. Patch de capteur selon la revendication 1, les conducteurs électriques étant intégrés dans le substrat non tissé.

3. Patch de capteur selon la revendication 1, les conducteurs électriques étant agencés sur une surface proximale (110A) du substrat non tissé.

4. Patch de capteur selon la revendication 1, les conducteurs électriques étant agencés sur une surface distale (110B) du substrat non tissé.

5. Patch de capteur selon l'une quelconque des revendications 1 à 4, une surface distale (110B) de la couche de détection étant la surface distale (100B) du patch de capteur.

6. Patch de capteur selon la revendication 5, la surface distale de la couche de détection étant configurée pour adhérer à la surface adhésive d'une plaque de base d'un appareil de stomie.

7. Patch de capteur selon l'une quelconque des revendications 1 à 6, les conducteurs électriques comprenant une pluralité d'électrodes, la pluralité d'électrodes comprenant une première électrode (121) et une seconde électrode (122).

8. Patch de capteur selon la revendication 7, la pluralité d'électrodes étant des traces conductrices imprimées sur une surface du substrat non tissé.

9. Patch de capteur selon l'une quelconque des revendications 1 à 8, le patch de capteur comprenant une interface de moniteur pour coupler électriquement un dispositif de moniteur aux conducteurs électriques.

10. Système de stomie comprenant un patch de capteur (10) selon l'une quelconque des revendications 1 à 9 et une plaque de base (20) d'un appareil de stomie, la surface distale du patch de capteur étant configurée pour être fixée à une surface adhésive de la plaque de base.
